Europäisches Patentamt

European Patent Office

Office européen des brevets

(19)

(11) Numéro de publication : **0 102 869**
**B1**

(12) FASCICULE DE BREVET EUROPÉEN

(45) Date de publication du fascicule du brevet :
22.10.86

(51) Int. Cl.⁴ : **H 05 G  1/52**, H 01 J 35/06,
A 61 B  6/02

(21) Numéro de dépôt : 83401535.6

(22) Date de dépôt : 26.07.83

(54) Tube à rayons X universel pour la stéréographie.

(30) Priorité : 06.08.82 FR 8213801

(43) Date de publication de la demande :
14.03.84 Bulletin 84/11

(45) Mention de la délivrance du brevet :
22.10.86 Bulletin 86/43

(84) Etats contractants désignés :
CH DE GB LI

(56) Documents cités :
FR-A-. 2 374 885
FR-A- 2 441 267
GB-A- 2 034 149
US-A- 1 610 863
US-A- 3 452 232
US-A- 3 591 821

(73) Titulaire : THOMSON-CSF
173, Boulevard Haussmann
F-75379 Paris Cedex 08 (FR)

(72) Inventeur : Gabbay, Emile
Thomson CSF SCPI 173, bid Hausmann
75379 Paris Cedex 08 (FR)

(74) Mandataire : Grynwald, Albert et al
THOMSON-CSF SCPI 19, avenue de Messine
F-75008 Paris (FR)

**0 102 869**

## Description

La présente invention concerne un tube à rayons X universel pour la stéréographie, utilisable dans le domaine de la radiologie, et plus particulièrement dans celui du radiodiagnostic.

L'effet stéréographique est obtenu en radiographiant un même sujet à partir de deux sources, localisées indépendamment dans l'espace, déterminant chacune une image ou cliché du sujet vu sous une incidence différente. Une vision stéréoscopique de ce sujet est obtenue en regardant ces deux images en vision binoculaire, c'est-à-dire que chacune des images est regardée indépendamment par chacun des deux yeux ; les deux perceptions rétiniennes sont envoyées par les nerfs optiques au cerveau, qui en fait la fusion et la perception en trois dimensions.

La qualité de cette vision en trois dimensions est liée aux conditions des prises des clichés, et aux conditions dans lesquelles ces clichés sont visionnés. Ainsi dans un premier cas, dans lequel des clichés sont pris avec un objet au voisinage ou au contact d'un film, déterminant un agrandissement minimum, une visualisation en trois dimensions reproduisant cet objet dans les meilleures conditions, est obtenue quand :

a) une distance entre les deux sources est égale à la distance entre les yeux, soit approximativement 65 millimètres ;

b) quand une distance « yeux-clichés » à la lecture, est égale à une distance « sources-clichés » à la prise de clichés.

Ceci pose un problème du fait qu'en pratique, pour une meilleure observation de détails, la distance « yeux-clichés » à la lecture est inférieure à la distance « sources-clichés » de la prise de clichés. Une modification des conditions de prises de clichés par exemple, effectuée dans un premier sens, est susceptible de rétablir la qualité de cette vision en trois dimensions ; mais cette modification doit s'exercer dans un sens contraire au premier, lorsque, afin d'obtenir un agrandissement plus important, l'objet est éloigné du film à la prise des clichés.

Une demande de brevet français publiée sous le N° 2 441 267, décrit un tube à rayons X pour la stéréographie. Ce tube permet d'établir sur une anode, successivement, soit deux sources ayant une même première dimension et constituant une première paire, soit deux autres sources ayant une même seconde dimension différente de la première. L'inconvénient d'un tel agencement réside en ce qu'un nombre n de paires de sources de rayonnement X exige un nombre n multiplié par deux des sources d'électrons.

Le tube à rayons X objet de la présente invention, permet la prise de clichés stéréographiques, visionnable à une distance courante dans la pratique en conservant la qualité de la vision en trois dimensions, tout en autorisant différentes valeurs d'agrandissement ; le tube selon l'invention étant agencé pour permettre d'une manière simple, de sélectionner deux sources parmi une pluralité de sources identiques.

Selon l'invention, un tube à rayons X universel pour la stéréographie, comportant une anode tournante et n cathodes, n étant au moins égal à trois, lesdites cathodes permettant d'établir sur une cible anodique, n sources de faisceaux de rayonnement X, caractérisé en ce que lesdites cathodes sont indépendantes et en ce que lesdites sources sont identiques et distantes les unes des autres d'une distance différente, lesdits faisceaux ayant de mêmes caractéristiques, deux quelconques d'entre ces sources étant associées afin de constituer un couple de travail choisi en fonction de la distance des sources qui le constituent, celles-ci étant établies alternativement afin que le faisceau de rayonnement X qu'elles génèrent permette une prise de clichés stéréographiques d'un sujet, compatible avec une valeur d'agrandissement choisie.

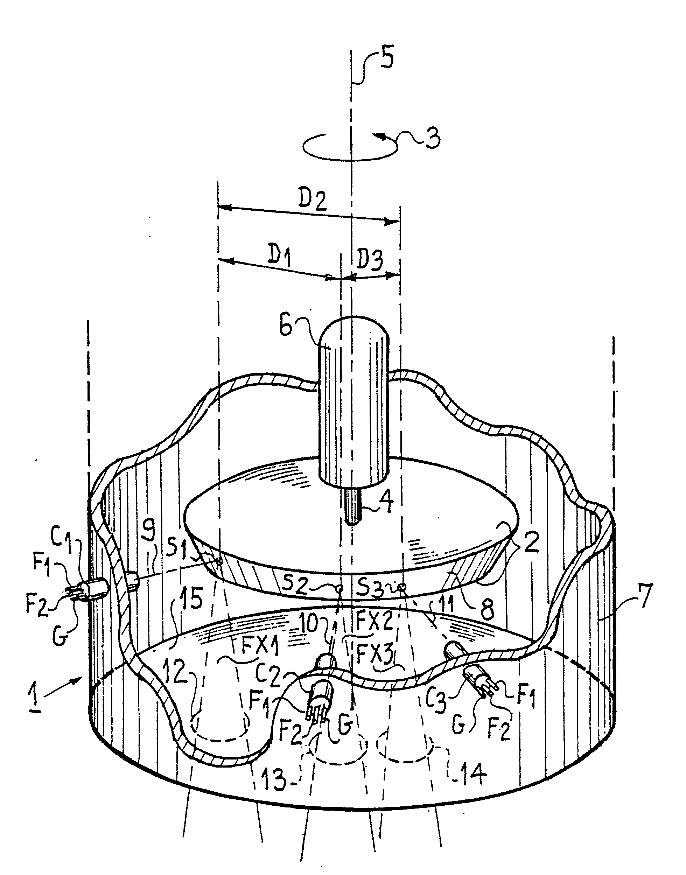

L'invention sera mieux comprise grâce à la description qui suit et de l'unique figure annexée.

Cette figure représente schématiquement, par une vue en perspective, des éléments caractéristiques d'un tube 1 à rayons X conforme à l'invention.

Le tube 1 comporte une anode tournante 2, entraînée en rotation selon une flèche 3 par exemple, autour d'un premier axe 5 perpendiculaire au plan de l'anode tournante 2 et passant par un centre 4 de celle-ci. Cette rotation est obtenue d'une manière classique par un moteur constitué d'un rotor 6 et d'un stator (non représenté) disposé de manière à tenir compte des problèmes d'isolation électrique et d'étanchéité bien connus de l'homme de l'art. L'anode tournante 2 a une forme tronconique, le tronc de cône constituant une cible anodique 8.

Une enveloppe 7, représentée partiellement pour une meilleure clarté du dessin, supporte une première, une seconde et une troisième cathode $C_1$, $C_2$, $C_3$, disposées de façon à être contenues dans le plan de rotation de l'anode tournante 2 ; ces cathodes sont capables chacune d'émettre un faisceau d'électrons (non représenté) selon un axe 9, 10, 11 également compris dans le plan de rotation de l'anode et, orienté de manière à intercepter le premier axe 5.

Un foyer formé par l'impact de chaque faisceau d'électrons sur la cible anodique 8, constitue une première, une seconde et une troisième source $S_1$, $S_2$, $S_3$, de mêmes caractéristiques, d'un faisceau de rayonnement X $FX_1$, $FX_2$, $FX_3$. Ces cathodes $C_1$, $C_2$, $C_3$ sont disposées de manière à ce que les sources $S_1$, $S_2$, $S_3$ qu'elles déterminent soient à des distances $D_1$, $D_2$, $D_3$ différentes les unes des autres.

Ceci constitue un exemple non limitatif d'un tube 1 selon l'invention, un tel tube pouvant comporter

2

un plus grand nombre de cathodes (non représentées) disposées de manière à permettre l'établissement d'autant de sources $S_1$, $S_2$, $S_3$, ..., $S_n$ d'un faisceau de rayonnement X $FX_1$, $FX_2$, $FX_3$, ..., $FX_n$, situées à des distances $D_1$, $D_2$, $D_3$, ..., $D_n$ différentes les unes des autres.

Ainsi qu'il a été précédemment décrit dans le préambule, une visualisation en trois dimensions exige à la prise des clichés, l'utilisation alternative d'une première et d'une seconde source de même nature, localisées en des points différents représentant une distance donnée, ces deux sources constituant un couple de travail. Dans un tube 1 selon l'invention, un tel couple de travail est obtenu par l'association de deux quelconques de ces trois sources $S_1$, $S_2$, $S_3$, le couple de travail ainsi constitué étant singularisé par une première ou une seconde ou une troisième distance $D_1$, $D_2$, $D_3$ entre les deux sources qui le constituent. Cette association de deux de ces sources $S_1$, $S_2$, $S_3$ est réalisée par une commande (non représentée) appliquée à celles des cathodes $C_1$, $C_2$, $C_3$, chargée d'établir respectivement une source $S_1$, $S_2$, $S_3$ de rayonnements X.

Pour cela, chaque cathode est indépendante et comporte d'une manière classique des éléments (non représentés) suivants :

— un filament générateur d'électrons, auquel est appliqué un courant de chauffage par l'intermédiaire d'une première et d'une seconde borne de sortie $F_1$, $F_2$ ;

— un élément appelé grille, autorisant ou non le passage des électrons vers l'anode tournante 2, auquel est appliquée, par l'intermédiaire d'une troisième borne de sortie G, la commande ci-dessus mentionnée. Cette commande consiste en une tension, polarisant la grille négativement par rapport au filament, afin de bloquer les électrons issus du filament ; la suppression de cette tension de polarisation autorise la formation du faisceau d'électrons.

Les cathodes $C_1$, $C_2$, $C_3$ sont ainsi indépendantes, pouvant être commandées indépendamment les unes des autres, et établir dans un ordre désiré les sources $S_1$, $S_2$, $S_3$ de rayonnements X : le filament de chaque cathode correspondant à un couple de travail pouvant être alimenté en permanence.

Ainsi un premier couple de travail peut être obtenu par l'association de la première et de la seconde source $S_1$, $S_2$, distantes de la première distance $D_1$ ; un second couple de travail formé par la première et la troisième source $S_1$, $S_3$, correspond à la seconde distance $D_2$, et un troisième couple formé par la seconde et la troisième source $S_2$, $S_3$, correspond à la troisième distance $D_3$.

Ceci constitue une caractéristique importante de l'invention qui permet d'obtenir dans l'exemple non limitatif décrit trois couples de travail $S_1$-$S_2$, $S_1$-$S_3$, $S_2$-$S_3$, alors qu'avec deux cathodes un seul couple de travail peut être obtenu.

Un tube 1, selon l'invention, peut comporter n cathodes $C_1$, $C_2$, $C_3$, ..., $C_n$ permettant d'obtenir un nombre de tels couples de travail, déterminé par une loi suivante :

$$C_n^2 = \frac{n!}{2!\,(n-2)!}$$

où $C_n^2$ représente le nombre de combinaisons par 2 possible pour n cathodes.

Ces couples de travail correspondant chacun à une distance $D_1$, $D_2$, $D_3$ différente, permettent chacun la prise de clichés stéréographiques d'un sujet (non représenté) avec une valeur d'agrandissement différente.

Le tableau suivant indique à titre d'exemple non limitatif la valeur des distances $D_1$, $D_2$, $D_3$ correspondant à chaque couple de travail $S_1$-$S_2$, $S_2$-$S_3$, $S_1$-$S_3$, ainsi que des valeurs d'agrandissement compatibles avec chaque couple de travail :

| COUPLE DE TRAVAIL | DISTANCE | AGRANDISSEMENT |
|---|---|---|
| 1er couple :<br>source $S_1$-$S_2$ | $D_1$ = 45 mm | Agrandissement moyen :<br>1,2 à 1,7 par exemple |
| Second couple :<br>Source $S_1$-$S_3$ | $D_2$ = 70 mm | Agrandissement nul<br>ou faible : objet en<br>contact du film ou<br>voisin du contact |
| Troisième couple<br>Source $S_2$-$S_3$ | $D_3$ = 20 mm | Agrandissement fort :<br>1,7 à 3 par exemple |

**0 102 869**

Dans l'exemple non limitatif de la description, l'enveloppe 7 est métallique, et les cathodes $C_1$, $C_2$, $C_3$ sont au même potentiel que cette enveloppe ; les faisceaux de rayonnements $FX_1$, $FX_2$, $FX_3$ émergent par des fenêtres de sorties 12, 13, 14 représentées en traits pointillés sur la figure, ces fenêtres de sortie étant disposées sur une face 15, de l'enveloppe 7, parallèle au plan de l'anode tournante 2. Il est à remarquer qu'une telle disposition permet de réaliser et d'utiliser un couple de travail formé par des sources (non représentées), établies sur la cible anodine 8 à des positions diamétralement opposées par rapport à l'anode tournante 2. L'alimentation du tube peut être du type monopolaire, ce qui facilite les commutations de cathodes $C_1$, $C_2$, $C_3$, celles-ci étant alors au potentiel de la terre et l'anode tournante 2 à la haute tension positive.

Un tube 1 à rayons X conforme à l'invention est du plus grand intérêt pour la stéréographie, grâce à ses multiples possibilités qui permettent de l'appliquer au radiodiagnostic, dans des cas tels que par exemple : vasculaire crânien, abdominal, cas de fractures fines.

**Revendications**

1. Tube à rayons X universel pour la stéréographie, comportant une anode tournante (2) et n cathodes ($C_1$, $C_2$, $C_3$, ..., $C_n$), n étant au moins égal à trois, lesdites cathodes ($C_1$, ..., $C_n$) permettant d'établir sur une cible anodique (8) n sources ($S_1$, $S_2$, $S_3$, ..., $S_n$) de faisceaux de rayonnement X ($FX_1$, $FX_2$, $FX_3$, ..., $FX_n$), caractérisé en ce que lesdites cathodes ($C_1$, ..., $C_n$) sont indépendantes et en ce que lesdites sources ($S_1$, ..., $S_n$) sont identiques et distantes les unes des autres d'une distance différente ($D_1$, $D_2$, $D_3$, ..., $D_n$), lesdits faisceaux X ($FX_1$, ..., $FX_n$) ayant de mêmes caractéristiques, deux quelconques d'entre ces sources étant associées afin de constituer un couple de travail ($S_1$, $S_2$ ou $S_1$, $S_3$ ou $S_2$, $S_3$) choisi en fonction de la distance ($D_1$, $D_2$, $D_3$, ..., $D_n$) des sources qui le constituent, celles-ci étant établies alternativement afin que le faisceau de rayonnement X ($FX_1$, $FX_2$, $FX_3$, ..., $FX_n$) qu'elles génèrent permette une prise de clichés stéréographiques d'un sujet, compatible avec une valeur d'agrandissement choisie.

2. Tube à rayons X selon la revendication 1, caractérisé en ce que la cible anodique (8) est un tronc de cône.

3. Tube à rayons X selon l'une des revendications précédentes, caractérisé en ce que chaque couple de travail ($S_1$-$S_2$, $S_1$-$S_3$, $S_2$-$S_3$, ..., $S_1$-$S_n$) permet de réaliser des clichés stéréographiques compatibles avec un agrandissement différent.

4. Tube à rayons X, selon la revendication 1, caractérisé en ce qu'il comporte en outre une enveloppe métallique (7), supportant les cathodes ($C_1$, $C_2$, $C_3$, ..., $C_n$)

5. Tube à rayons X selon la revendication précédente, caractérisé en ce que les cathodes ($C_1$, $C_2$, $C_3$, ..., $C_n$) sont à un même potentiel que l'enveloppe (7) métallique.

6. Tube à rayons X selon la revendication 1, caractérisé en ce que les cathodes indépendantes ($C_1$, $C_2$, $C_3$, ..., $C_n$) sont disposées de façon à être contenues sensiblement dans un même plan que le plan de rotation de l'anode tournante (2), et que des fenêtres de sortie (12, 13, 14) du rayonnement X sont disposées sur une face de l'enveloppe parallèle au plan de l'anode tournante (2).

**Claims**

1. Universal X-ray tube for stereography comprising a rotary anode (2) and n cathodes ($C_1$, $C_2$, $C_3$, ..., $C_n$), n being at least equal to three, said cathodes ($C_1$, $C_2$, $C_3$, ..., $C_n$), n being at least equal to three, said cathodes ($C_1$, ..., $C_n$) enabling the establishment on an anode target (8) of n sources ($S_1$, $S_2$, $S_3$, ..., $S_n$) of radiation beams ($FX_1$, $FX_2$, $FX_3$, ..., $FX_n$), characterized in that said cathodes ($C_1$, ..., $C_n$) are independent of each other and that said sources ($S_1$, ..., $S_n$) are identical and spaced a different distance ($D_1$, $D_2$, $D_3$, ..., $D_n$) from each other, said X-ray beams ($FX_1$, ..., $FX_n$) having the same characteristics, any two of said sources being associated to constitute a working pair ($S_1$, $S_2$ or $S_1$, $S_3$ or $S_2$, $S_3$) chosen as a function of the distance ($D_1$, $D_2$, $D_3$, ..., $D_n$) between the sources constituting it, the latter being activated alternately so that the X-ray beam ($FX_1$, $FX_2$, $FX_3$, ..., $FX_n$) generated thereby permits stereographic shots compatible with a selected magnification to be made of a subject.

2. X-ray tube according to claim 1, characterized in that the anode target (8) is a cone trunk.

3. X-ray tube according to any one of the preceding claims, characterized in that each working pair ($S_1$-$S_2$, $S_1$-$S_3$, $S_2$-$S_3$, ..., $S_1$-$S_n$) permits the realization of stereographic shots compatible with different magnification.

4. X-ray tube according to claim 1, characterized in that it further includes a metallic envelope (7) carrying the cathodes ($C_1$, $C_2$, $C_3$, ..., $C_n$).

5. X-ray tube according to the preceding claim, characterized in that the cathodes ($C_1$, $C_2$, $C_3$, ..., $C_n$) lie at the same potential as the metallic envelope (7).

6. X-ray tube according to claim 1, characterized in that the independent cathodes ($C_1$, $C_2$, $C_3$, ..., $C_n$) are arranged so as to be contained substantially in the same plane as the plane of rotation of the rotary anode (2) and that the outlet windows (12, 13, 14) for the X-ray radiation are disposed on a face of the envelope parallel to the plane of the rotary anode (2).

4

**Patentansprüche**

1. Universelle Röntgenröhre für die Stereographie, versehen mit einer Drehanode (2) und n Kathoden ($C_1$, $C_2$, $C_3$, ..., $C_n$), wobei n wenigstens gleich drei ist und die genannten Kathoden ($C_1$, ..., $C_n$) die Herstellung von n Quellen ($S_1$, $S_2$, $S_3$, ..., $S_n$) von Röntgenstrahlungsbündeln ($FX_1$, $FX_2$, $FX_3$, ..., $FX_n$) auf einem Anodentarget (8) ermöglichen, dadurch gekennzeichnet, daß die genannten Kathoden ($C_1$, ..., $C_n$) voneinander unabhängig sind und daß die genannten Quellen ($S_1$, ..., $S_n$) einander völlig gleich sind sowie voneinander verschiedene Abstände ($D_1$, $D_2$, $D_3$, ..., $D_n$) aufweisen, wobei die genannten Röntgenbündel ($FX_1$, ..., $FX_n$) gleiche Kenndaten aufweisen, wobei ferner zwei beliegibe Quellen einander zugeordnet sind, um ein Arbeitspaar ($S_1$, $S_2$ oder $S_1$, $S_3$ oder $S_2$, $S_3$) zu bilden, welches in Abhängigkeit von dem Abstand ($D_1$, $D_2$, $D_3$, ..., $D_n$) zwischen den dieses bildenden Quellen gewählt ist, und wobei letztere abwechselnd aktiviert werden, damit das von ihnen erzeugte Röntgenstrahlungsbündel ($FX_1$, $FX_2$, $FX_3$, ..., $FX_n$) ermöglicht, daß stereographische, mit einem gewählten Vergrößerungswert kompatible Aufnahmen von einem Gegenstand gemacht werden können.

2. Röntgenröhre nach Anspruch 1, dadurch gekennzeichnet, daß das Anodentarget (8) ein Kegelstumpf ist.

3. Röntgenröhre nach einem der vorstehenden Ansprüche, dadurch gekennzeichnet, daß jedes Arbeitspaar ($S_1$-$S_2$, $S_1$-$S_3$, $S_2$-$S_3$, ..., $S_1$-$S_n$) die Verwirklichung von stereographischen Aufnahmen ermöglicht, die mit verschiedener Vergrößerung kompatibel sind.

4. Röntgenröhre nach Anspruch 1, dadurch gekennzeichnet, daß sie ferner eine metallische Hülle (7) umfaßt, welche die Kathoden ($C_1$, $C_2$, $C_3$, ..., $C_n$) trägt.

5. Röntgenröhre nach dem vorstehenden Anspruch, dadurch gekennzeichnet, daß die Kathoden ($C_1$, $C_2$, $C_3$, ... $C_n$) auf demselben Potential wie die metallische Hülle (7) liegen.

6. Röntgenröhre nach Anspruch 1, dadurch gekennzeichnet, daß die voneinander unabhängigen Kathoden ($C_1$, $C_2$, $C_3$, ..., $C_n$) derart angeordnet sind, daß sie in etwa in derselben Ebene wie die Drehebene der Drehanode (2) enthalten sein können, und daß die Austrittsfenster (12, 13, 14) für die Röntgenstrahlung auf einer Fläche der Umhüllung, die parallel zur Ebene der Drehanode (2) liegt, angeordnet sind.